# DEMANDE DE BREVET EUROPEEN

(11) **EP 4 258 272 A1**
(43) Date de publication de la demande: **11.10.2023**
(21) Numéro de dépôt: 23161766.3
(22) Date de dépôt: 14.03.2023
(51) Int. Cl.: G16H 10/60, G16H 20/70, G16H 40/63, G16H 40/67, G16H 50/20, G16H 50/30, G16H 50/70, A61B 5/02, A61B 5/021, A61B 5/00, G06N 3/04, G06N 3/08, G06N 7/00, G06N 20/20, A61B 5/01, A61B 5/0531, A61B 5/11

(54) **PROCÉDÉ DE LABELLISATION DE DONNÉES POUR CONSTRUIRE UNE BASE DE DONNÉES POUR CONFIGURER, VALIDER ET/OU TESTER UNE APPLICATION DE SURVEILLANCE DU NIVEAU DE FATIGUE D'UN INDIVIDU**

(30) Priorité: 15.03.2022 FR 2202257
(71) Demandeur: THALES, 92400 Courbevoie (FR)
(72) Inventeur: BERTHELOT, Bastien, 31036 TOULOUSE (FR)
(74) Mandataire: Lavoix

(57) **Abrégé**

Ce procédé (100) comporte les étapes de : acquisition (110) de données utiles (D1) au cours d'une phase d'expérimentation, les données utiles étant des données physiologiques obtenues au moyen de capteur(s) et qui correspondent chacune à une donnée d'entrée de l'application de surveillance ; acquisition (110) de données déclaratives (D2) de l'individu, en temps réel durant la phase d'expérimentation et/ou en temps différé avant et/ou après la phase d'expérimentation ; fusion (120) des données utiles et des données déclaratives de manière à calculer un niveau de fatigue vrai (EV) ; labellisation (130) des données utiles avec le niveau de fatigue vrai calculé ; et stockage (140) des données utiles labellisées dans la base de données d'apprentissage.

## Description

La présente invention a pour domaine général celui des moyens permettant d'évaluer, en temps réel, un niveau de fatigue d'un individu, par exemple le niveau de fatigue d'un pilote d'un aéronef, ou celui d'un contrôleur aérien travaillant devant une console de visualisation radar, ou encore celui d'un conducteur d'un véhicule automobile ou ferroviaire.

En effet, lorsque le niveau de fatigue, d'un pilote d'aéronef par exemple, devient trop important, sa vigilance se réduit fortement au point de ne plus pouvoir effectuer les tâches nécessaires à la poursuivre du vol en sécurité.

Un état comme la somnolence étant généralement non-conscient, et donc non détectable par le pilote lui-même, il est utile de mettre en oeuvre des procédés automatiques de surveillance permettant d'évaluer le niveau de fatigue instantané du pilote et de déclencher une contremesure lorsqu'il est détecté que ce niveau de fatigue relève d'un niveau critique.

On connaît notamment des applications de surveillance physiologiques mettant en oeuvre des algorithmes d'analyse de certaines grandeurs physiologiques pertinentes du pilote sous surveillance, collectées au moyen de différents capteurs. On peut citer l'exemple d'un électroencéphalogramme de mesure de l'activité cérébrale du pilote permettant d'évaluer, en temps réel, son niveau de fatigue.

De tels application de surveillance sont calibrées et/ou validées et/ou testées en utilisant des jeux de données labellisées (ou étiquetées), c'est-à-dire des jeux de données pour lesquels on connaît le niveau de fatigue vrai que ces données décrivent (notion de « vérité terrain »). Il s'agit alors d'utiliser ces jeux de données labellisées pour ajuster les paramètres de l'application de surveillance de manière à ce que le niveau de fatigue évalué par la mise en oeuvre de l'application de surveillance une fois correctement paramétrée corresponde au mieux au niveau de fatigue vrai.

Il apparaît ainsi que la labellisation d'un jeu de données est une étape cruciale pour que le procédé de surveillance soit configuré de manière à être efficace.

Actuellement la labellisation des données est effectuée par des experts. Elle requière un temps d'analyse long. La labellisation a donc un coût élevé.

De plus, puisqu'ils résultent d'un processus réalisé par des experts, les jeux de données labellisées obtenus sont peu nombreux, présentent un nombre de données faible et une variabilité réduite. Ils ne permettent donc pas un entrainement robuste des procédés de surveillance.

Il y a donc un besoin d'automatiser la labellisation de données, ce qui permettra de l'étendre pour traiter des données ayant un plus grand nombre de composantes afin de mieux décrire le niveau de fatigue de l'individu surveillé, et pour qualifier un plus grand nombre de données de manière à couvrir un plus grand nombre de situations. Les jeux de données labellisées obtenus permettront aux applications de surveillance d'être entrainées de manière plus robuste afin d'estimer avec plus d'exactitude le niveau de fatigue instantané de l'individu surveillé.

L'article de LI JUE et al, "Identification and classification of construction equipment operator's mental fatigue using wearable eye-tracking technology", AUTOMATION IN CONSTRUCTION, ELSEVIER, AMSTERDAM, NL, vol. 109, 31 octobre 2019, D1 permet d'évaluer automatiquement la fatigue mentale d'un opérateur de manière non-invasive en utilisant le suivi des mouvements oculaires. Selon ce document, il est prévu une première étape de collecte de données utiles, une seconde étape d'identification automatique d'une pluralité de niveaux de fatigue, une troisième étape de labellisation automatique des données utiles avec un niveau de fatigue, et une quatrième étape d'entrainement d'un algorithme de classification dont les entrées correspondent aux données utiles.

La présente invention a donc pour objet un procédé de labellisation de données alternatif permettant d'apposer un label du type niveau de fatigue sur des données collectées en situation réelle, lors d'un vol d'entrainement ou lors d'une cession en simulateur.

Pour cela l'invention a pour objet un procédé de labellisation de données et un produit programme d'ordinateur pour mettre en oeuvre le procédé de labellisation précédent.

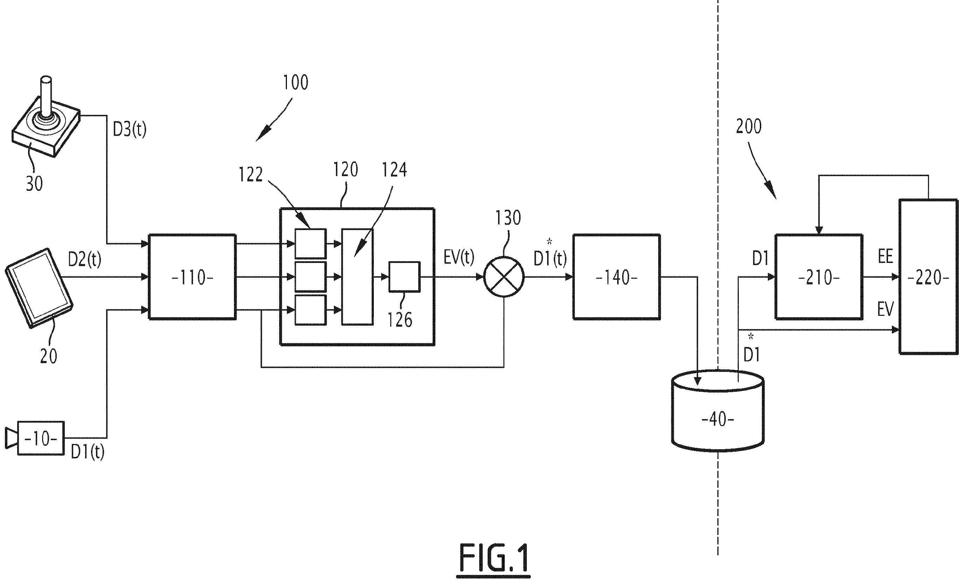

L'invention et ses avantages seront mieux compris à la lecture de la description détaillée qui va suivre d'un mode de réalisation particulier, donné uniquement à titre d'exemple non limitatif, cette description étant faite en se référant à l'unique figure [Fig 1] annexée qui représente, sous forme de blocs, un mode de réalisation préféré du procédé de labellisation selon l'invention.

Un mode de réalisation privilégié du procédé de labellisation selon l'invention va être présenté en référence à la figure annexée.

De préférence, le procédé de labellisation selon l'invention est mis en oeuvre sous forme d'un logiciel exécuté par un ordinateur. Cet ordinateur comporte des moyens de calcul, tels qu'un processeur, des moyens de mémorisation, tels qu'une mémoire, et des moyens d'interface, tels que des ports d'entrée/sortie de connexion à un ou plusieurs capteurs, une ou plusieurs interfaces homme-machine ou un ou plusieurs systèmes desquels l'ordinateur reçoit des données à fusionner. Les moyens de mémorisation stockent les instructions de programmes d'ordinateurs, en particulier un programme dont l'exécution permet la mise en oeuvre du procédé selon l'invention.

Sur cette figure, le procédé de labellisation 100 permet de renseigner automatiquement une base de données d'apprentissage 40, à partir des données labellisées de laquelle pourra être mis en oeuvre un procédé de configuration 200 d'une application de surveillance du niveau de fatigue d'un pilote.

### Application de surveillance

Dans le présent mode de réalisation, l'application de surveillance, une fois convenablement configurée, est utilisée pour la surveillance en temps réel du niveau de fatigue d'un pilote se trouvant dans un poste de commande d'un aéronef, que ce poste de commande soit embarqué à bord de l'aéronef ou déporté dans le cas d'un drone par exemple.

Cette application de surveillance s'appuie sur un premier ensemble de données physiologiques, dites données utiles, en tant que données d'entrée de l'application permettant d'estimer le niveau de fatigue instantané du pilote.

Ces données utiles sont acquises par un ou plusieurs capteurs prévus soit sur le pilote lui-même (cas d'un capteur porté), soit dans le poste de commande de l'aéronef (capteur déporté).

Chaque capteur est configuré pour mesurer au moins une grandeur physiologique instantanée du pilote, ayant un lien avec le niveau de fatigue de ce dernier.

Eventuellement, les données brutes délivrées par un capteur subissent un prétraitement afin d'élaborer des données utiles pouvant être appliquée en entrée de l'application de surveillance.

Le traitement en temps réel des données utiles acquises permet à l'application de surveillance d'estimer un niveau de fatigue instantané du pilote et avantageusement de déclencher automatiquement une réponse adaptée dès lors que ce niveau de fatigue est jugé incompatible avec la mission du pilote.

Par exemple, l'application de surveillance est propre à classer l'état du pilote caractérisé par les données utiles soit dans une classe « fatigue nominale », soit dans un classe « fatigue dangereuse ». En variante ou en combinaison, l'application de surveillance est propre à quantifier le niveau de fatigue de l'état du pilote avec une échelle continue, par exemple graduée sur une centaine de points.

### Généralités

Le procédé de labellisation 100 est fondé sur le traitement de données de différentes modalités (ou natures) pour la détermination d'un niveau de fatigue instantané vrai (ou terrain).

Le procédé de labellisation 100 comporte une première étape 110 d'acquisition de données, une seconde étape 120 de fusion de données, une troisième étape 130 de labellisation de données avec un niveau de fatigue vrai, et une quatrième étape 140 de stockage des données ainsi labellisées dans la base de données d'apprentissage 40.

### Etape 110

Les données acquises au cours de l'étape 110 sont de deux types : il y a d'abord les données utiles formant un premier ensemble de données et qui constituent les entrées de l'application de surveillance ; il y a ensuite les données de labellisation formant un second ensemble de données et qui ne constituent pas des entrées de l'application de surveillance, mais qui sont utilisées dans le procédé de labellisation pour mieux décrire l'état du pilote et permettre de déterminer le niveau de fatigue vrai avec plus d'exactitude.

La première étape 110 est mise en oeuvre au cours d'un vol expérimental pour reproduire au mieux les conditions réelles dans lesquelles l'application de surveillance sera mise en oeuvre.

Cette première étape 110 vise à collecter des données des différentes modalités :
Il s'agit d'abord de collecter les données utiles butes D1. Les moyens pour acquérir ce premier ensemble de données sont référencés de manière générale par le chiffre 10 sur la figure. Il s'agit d'utiliser des capteurs identiques ou similaires à ceux utilisés lors de la mise en oeuvre du l'application de surveillance.

Les capteurs utilisés sont de préférence peu ou faiblement invasifs, pour mesurer en continu une ou plusieurs grandeurs physiologiques pertinentes. Il s'agit par exemple de mettre en oeuvre une caméra d'observation du visage du pilote pour obtenir, après un prétraitement adapté, réalisé de préférence a posteriori, une activité oculaire instantanée ou encore une gestuelle du pilote en réponse à un stimulus donné (situation opérationnelle, information affichée sur une interfaces homme/machine). Il s'agit par exemple de mettre en oeuvre un capteur de mesure de la fréquence cardiaque instantanée du pilote, tel qu'une montre connectée. Il s'agit par exemple de mettre en oeuvre un capteur intégré à un bouchon d'oreille pour la mesure de la température et du taux d'oxygénation du sang du pilote.

Il s'agit ensuite de collecter des données de labellisation brutes D2. Les moyens pour acquérir ce second ensemble de données sont référencés de manière générale par le chiffre 20 sur la figure.

Les données de labellisation sont des grandeurs physiologiques qui sont très fortement corrélées à l'état neuropsychologique du pilote. Cependant, la mesure de ces grandeurs fait appel à des capteurs qui ne peuvent être mis en oeuvre dans un cas réel, car trop invasifs et ils seraient alors difficilement accepté par le pilote en vol, ou peu robuste et ils ne supporteraient pas d'être employés dans les conditions très variables des opérations aéronautiques.

En particulier, les capteurs utilisés pour l'acquisition des données de labellisation sont choisis dans le groupe constitué de : un capteur cardiaque, notamment un électrocardiographe ; un oxymètre de pouls, notamment un capteur à photopléthysmographie ; un capteur de respiration ; un accéléromètre ; une électrode crânienne, par exemple un électroencéphalographe ; un capteur de pression agencé dans le siège de l'opérateur ; un capteur de pression agencé dans un dispositif de commande propre à être actionné par l'opérateur ; un capteur de sudation de l'opérateur ; un capteur de réponse électrodermale ; une caméra configurée pour capter au moins une image comprenant au moins une partie de l'opérateur, notamment les yeux pour de l'oculométrie (ou « eye tracking » en anglais) ; un microphone ; un capteur infrarouge de température de peau de l'opérateur ; un capteur de température interne de l'opérateur ; et un bandeau à spectroscopie proche infrarouge (aussi appelé bandeau « fNIRS ») utilisant la lumière proche infrarouge pour surveiller l'activité du cerveau.

Les données de labellisation comportent avantageusement également des données déclaratives, obtenues au moyen d'une ou plusieurs interfaces adaptées permettent d'interroger le pilote sur son ressenti, en temps réel durant le vol expérimental et/ou en temps différé avant et/ou après le vol expérimental. Il s'agit de données déclaratives subjectives discrètes et acquises à faible fréquence pour ne pas gêner le pilote durant le vol. Avant le vol, les données déclaratives saisies par le pilote peuvent par exemple permettre de définir une catégorie générale dont relève le pilote ou encore de définir le scénario opérationnel que le vol expérimental va permettre de tester. On peut citer par exemple l'échelle de Karolinska Sleepiness Scale (KSS), l'échelle de Samn-Perelli à sept niveaux de fatigue (SPS), une échelle continue et visuelle de fatigue, ou encore des éléments propres au sommeil (temps d'éveil, heure de sommeil sur les trois dernières nuits, etc.)

Avantageusement, il s'agit également de collecter des données environnementales D3. Les moyens pour acquérir ce troisième ensemble de données sont référencés de manière générale par le chiffre 30 sur la figure. Ces données sont relatives à l'environnement dans lequel se déroule le vol, les actions du pilote et ses performances dans la gestion des systèmes dont est équipé l'aéronef. Il s'agit par exemple d'enregistrer les actions de commande réalisées par le pilote sur les systèmes de pilotage de l'aéronef, ou les temps de réponse à l'exécution d'une tâche suite à l'occurrence d'un évènement particulier au cours du vol. Il s'agit par exemple encore d'enregistrer les erreurs ou les manquements d'alarmes. Il s'agit d'une acquisition en continu. Même si certains enregistrements sont discrets, les moyens d'acquisition correspondant sont en permanence en veille.

Les données brutes acquises par chacun des dispositif 10, 20 et 30 sont datées avec leur instant t d'acquisition. Les données D1(t), D2(t) et D3(t) sont de préférence enregistrées dans la base de données d'apprentissage 40 à chaque instant du vol expérimental.

### Etape 120

Le procédé de labellisation 100 se poursuit par une seconde étape 120 de fusion des données acquises lors de l'étape 110 et relatives à un même vol expérimental.

Cette seconde étape est de préférence réalisée hors ligne, c'est-à-dire après le vol.

Elle permet d'analyser les données brutes stockées dans la base de données d'apprentissage 40 en vue de construire un jeu de données labellisées.

De préférence, dans une première étape élémentaire, 122, chaque type de données brutes subit un prétraitement adapté. En particulier, les données utiles brutes sont prétraitées pour obtenir des données utiles dont le format correspond à celles appliquées en entrée de l'application de surveillance. Par exemple, les images acquises par la caméra sont analysées pour extraire des mouvements oculaires instantanés du pilote.

De plus, à l'étape 122, les données sont synchronisées au sens où un pas de temps est défini et les données sont datées par rapport à ce nouvel échantillonnage temporelle. Si une donnée a une fréquence d'échantillonnage supérieure à ce pas de temps, une moyenne de la valeur de la donnée est prise en compte. Si une donnée a une fréquence d'échantillonnage inférieure à ce pas de temps, la valeur de la donnée est répétée à chaque pas de temps, entre deux mises à jour de cette donnée.

Pour chaque pas de temps, on obtient ainsi un premier vecteur de données comportant les données utiles D1, et un second vecteur de données comportant les données de labellisation D2 et avantageusement les données d'environnement D3.

L'utilisation de données des différentes modalités D2 et D3, permet d'avoir une description globale de l'état du pilote et de l'environnement.

L'étape 120 se poursuit par une étape élémentaire 124 d'analyse permettant de déterminer, pour chaque second vecteur de données, la meilleure valeur possible du niveau de fatigue en tant que niveau de fatigue vrai EV pour le pas de temps considéré. Il s'agit donc d'une grandeur instantanée. C'est une information continue obtenue sur l'ensemble du vol expérimental.

Les valeurs possibles du niveau de fatigue vrai EV sont celles de la sortie de l'application de surveillance à configurer. Par exemple ces valeurs possibles appartiennent à l'ensemble comportant au moins deux catégories : un niveau de fatigue normal et un niveau de fatigue dangereux. En variante, l'ensemble des valeurs possibles comporte plus de deux classes. Par exemple, en plus des deux niveaux précédents, l'ensemble comporte un niveau de somnolence, un niveau de sommeil et un niveau d'évanouissement. Par exemple encore, la classification s'effectue selon trois niveaux de fatigue respectivement faible, modéré, et élevé.

Dans une autre variante, pouvant avantageusement être mise en oeuvre en parallèle des précédentes, la valeur possible du niveau de fatigue vrai EV est une grandeur continue par exemple entre 0 et 100, correspondant par exemple à la probabilité que l'état du pilote corresponde au niveau de fatigue dangereux.

Pour l'étape élémentaire 124 d'analyse, est avantageusement exécuté un algorithme d'apprentissage supervisé permettant de déterminer les valeurs possibles du niveau de fatigue vrai à partir des seconds vecteurs de données (et éventuellement des troisièmes vecteurs de données) collectés durant l'ensemble du vol d'entrainement.

Un tel algorithme d'apprentissage supervisé est par exemple du type réseaux de neurones, SVM (« Support Vector Machines » pour machines à vecteurs de support), KNN (« k-nearest neighbors » pour k plus proches voisins), régression logistique, etc. Les données de labellisation D2 et éventuellement d'environnement D3, constituent ainsi les données d'entrée de l'algorithme d'apprentissage supervisé. Dans domaine de l'intelligence artificielle, ces données d'entrée sont appelées communément « features » en anglais.

En variante, l'algorithme exécuté est : un algorithme d'apprentissage non supervisé, du type regroupement hiérarchique, k-means (pour k-moyennes), mélanges gaussiens, carte auto-organisatrice, etc. ; ou un algorithme de filtrage optimal, du type filtrage de Kalman et/ou filtrage particulaire ; ou encore un algorithme de modélisation non biaisée empirique, du type arbre de décision, etc.

Avantageusement, l'étape 120 comporte une étape élémentaire 126 de filtrage du niveau de fatigue vrai EV. Il s'agit par exemple de comparer le niveau de fatigue à l'instant courant t avec les niveaux de fatigue aux instants précédents pour éviter les évolutions trop rapides et donc erronées de cette variable. Par exemple le niveau de fatigue à l'instant courant t est pondéré par une moyenne des niveaux de fatigue aux instants précédents sur une fenêtre temporelle de largeur prédéfinie.

On obtient en sortie de l'étape 120, pour chaque pas de temps, un niveau de fatigue vrai EV.

La dynamique de labellisation, c'est-à-dire le pas de temps choisi pour l'étape de fusion, peut être adapté en fonction de l'application de surveillance. Par exemple, pour la détection d'un niveau de fatigue correspondant à une somnolence, qui est un processus lent, la dynamique de labellisation peut être plus lente, en choisissant un pas de temps plus long. Par exemple, si on souhaite avoir une valeur du niveau de fatigue toutes les secondes, le pas d'échantillonnage de l'étape 120 est de 1Hz.

### Etape 130

A l'étape 130, pour labéliser les données utiles à l'instant t, c'est-à-dire le premier vecteur de données à l'instant t, une corrélation temporelle est établie avec les données de labellisation à l'instant t, c'est-à-dire le second vecteur de données à l'instant t, et la valeur du niveau de fatigue vrai EV calculée pour ce second vecteur de données à l'instant t lors de l'étape 120 est associée aux données utiles D1 à l'instant t pour constituer des données utiles labellisées D*1 à l'instant t, c'est-à-dire un premier vecteur de données labellisées à l'instant t.

### Etape 140

Enfin, à l'étape 140, les données utile labellisées D*1 à l'instant t sont enregistrées dans la base de données d'apprentissage 40.

### Procédé de configuration 200

Le procédé de configuration 200 consiste à entrainer l'application de surveillance.

A l'étape 210, l'application de surveillance, configurée avec un premier jeu de paramètres de configuration, est exécutée sur chaque vecteur de données labellisées d'un jeu de vecteurs extrait de la base 40 pour obtenir une estimation du niveau de fatigue EE de ce vecteur.

A l'étape 220, le niveau de fatigue estimé EE est comparé avec la valeur du niveau de fatigue vrai EV pour chaque vecteur du jeu vecteurs utilisé à l'étape 210. Cette étape de comparaison conduit à une mise à jour des paramètres de configuration de l'application de surveillance.

L'itération des étapes 210 et 220 sur tout ou partie des données utiles labellisées D*1 de la base 40 permet d'optimiser les paramètres de configuration de l'application de surveillance.

Ce processus itératif est stoppé lorsqu'un critère de convergence des paramètres de configuration est vérifié, ou, plus simplement, après un certain nombre d'itérations du processus itératif. L'application de surveillance est alors considérée comme configurée.

En variante, au lieu d'être totalement disjoints, une partie du second ensemble de données de labellisation recoupe le premier ensemble de données utiles.

### Avantages

Le procédé de labellisation selon l'invention permet de labéliser des données acquises durant un vol en répondant aux critères suivants :
- Il permet de collecter des données de manière automatique dans un environnement similaire ou identique à celui où l'application de surveillance cible sera mise en oeuvre ;
- Il permet de déterminer finement le niveau de fatigue vrai à l'instant t à associer aux données utiles, mais en utilisant des données de labellisation différentes des données utiles. Ainsi, l'application de surveillance n'a pas besoin de prendre en entrée ces données de labellisation, et se limite à prendre en compte qu'un petit nombre de données utiles. La mise en oeuvre de l'application de surveillance dans un cas réel ne nécessite pas d'équiper le cockpit et/ou le pilote de capteurs complexes. Cela perturbe donc moins les activités du pilote pendant la phase de mise en oeuvre de l'application de surveillance ;
- Il permet de collecter des données de manière continue sur l'ensemble de l'expérimentation, et ceci à l'inverse des questionnaires et tests d'attention ;
- Il présente une plus grande objectivité que les méthodes de labellisation fondées sur les seules déclarations du pilote.

La base de données construite en mettant en oeuvre le présent procédé de labellisation peut servir non pas à entrainer l'application cible, mais à la valider ou à la tester.

## Revendications

1. Procédé (100) de labellisation de données utiles (D*1) afin de construire une base de données (40) permettant de configurer, valider et/ou tester une application de surveillance du niveau de fatigue d'un individu, **caractérisé en ce qu'**il comporte les étapes de :
- acquisition (110) de données utiles (D1) au cours d'une phase d'expérimentation, les données utiles étant des données physiologiques obtenues au moyen d'un ou plusieurs capteur(s) et qui correspondent chacune à une donnée d'entrée de l'application de surveillance ;
- acquisition (110) de données de labellisation (D2), en temps réel durant la phase d'expérimentation et/ou en temps différé avant et/ou après la phase d'expérimentation ;
- fusion (120) des données utiles et des données de labellisation et calcule d'un niveau de fatigue vrai (EV) pour chaque instant de la phase d'expérimentation, à partir des données de labellisation ;
- labellisation (130) des données utiles acquises à un instant avec le niveau de fatigue vrai calculé pour les données de labellisation acquises audit instant ; et,
- stockage (140) des données utiles labellisées dans la base de données d'apprentissage.

2. Procédé selon la revendication 1, comportant une étape supplémentaire d'acquisition de données d'environnement (D3), l'étape de fusion utilisant les données d'environnement en plus des données de labellisation pour calculer le niveau de fatigue vrai (EV) pour chaque instant de la phase d'expérimentation.

3. Procédé selon l'une quelconque des revendications 1 à 2, dans lequel le niveau de fatigue vrai est sélectionné parmi au moins deux niveaux.

4. Procédé selon l'une quelconque des revendications 1 à 3, dans lequel un capteur utilisé pour l'acquisition de données de labellisation est sélectionné parmi : un capteur cardiaque, notamment un électrocardiographe ; un oxymètre de pouls, notamment un capteur à photopléthysmographie ; un capteur de respiration ; un accéléromètre ; une électrode crânienne, par exemple un électroencéphalographe ; un capteur de pression agencé dans le siège de l'opérateur ; un capteur de pression agencé dans un dispositif de commande propre à être actionné par l'opérateur ; un capteur de sudation de l'opérateur ; un capteur de réponse électrodermale ; une caméra configurée pour capter au moins une image comprenant au moins une partie de l'opérateur, notamment les yeux pour de l'oculométrie; un microphone ; un capteur infrarouge de température de peau de l'opérateur ; un capteur de température interne de l'opérateur ; et un bandeau à spectroscopie proche infrarouge.

5. Procédé selon l'une quelconque des revendications 1 à 4, dans lequel l'étape de fusion consiste à exécuter :
- un algorithme d'apprentissage supervisé, du type réseaux de neurones, machines à vecteurs de support, k plus proches voisins, ou régression logistique ;
- un algorithme d'apprentissage non supervisé, du type regroupement hiérarchique, k moyennes, mélanges gaussiens, ou carte auto-organisatrice ;
- un algorithme de filtrage optimal, du type filtrage de Kalman et/ou filtrage particulaire ; ou
- un algorithme de modélisation non biaisée empirique, du type arbre de décision.

6. Procédé selon l'une quelconque des revendications 1 à 5, dans lequel, les données acquises étant des données brutes, l'étape de fusion comporte une étape élémentaire préalable de prétraitement des données brutes pour obtenir des données traitées.

7. Procédé selon la revendication 6, dans lequel, les données acquises étant des données brutes, l'étape de fusion comporte une étape élémentaire de synchronisation des données brutes en fonction du pas de temps choisi.

8. Procédé selon l'une quelconque des revendications 1 à 7, dans lequel, l'étape de fusion comporte une étape élémentaire de filtrage du niveau de fatigue vrai calculé pour chaque pas de temps.

9. Programme d'ordinateur comportant des instructions logicielles qui, lorsqu'elles sont exécutées par un ordinateur, mettent en oeuvre un procédé de labellisation selon la revendication précédente.
